# EUROPÄISCHE PATENTSCHRIFT

(11) **EP 4 151 187 B1**
(45) Veröffentlichungstag und Bekanntmachung des Hinweises auf die Patenterteilung: **05.11.2025**
(21) Anmeldenummer: 22196210.3
(22) Anmeldetag: 16.09.2022
(51) Int. Cl.: A61F 9/007, A61M 1/00, A61M 25/00, A61M 39/08

(54) **SCHLAUCH FÜR EIN MEDIZINISCHES BEHANDLUNGSSYSTEM SOWIE MEDIZINISCHES BEHANDLUNGSSYSTEM**
TUBE FOR A MEDICAL TREATMENT SYSTEM AND MEDICAL TREATMENT SYSTEM
TUYAU POUR SYSTÈME DE TRAITEMENT MÉDICAL ET SYSTÈME DE TRAITEMENT MÉDICAL

(30) Priorität: 21.09.2021 DE 102021124400
(43) Veröffentlichungstag der Anmeldung: 22.03.2023
(73) Patentinhaber: Carl Zeiss Meditec AG, 07745 Jena (DE)
(72) Erfinder: Werner, Andreas, 73431 Aalen (DE)
(74) Vertreter: Hofstetter, Schurack & Partner

(56) Entgegenhaltungen:
- EP-A2- 1 917 987
- US-A1- 2010 056 991

## Beschreibung

Die Erfindung betrifft einen Schlauch für ein medizinisches Behandlungssystem, insbesondere ein ophthalmochirurgisches System zur Behandlung eines Auges, wobei der Schlauch einen ersten Kanal zum Zuführen eines Irrigationsfluids von einer Konsole des medizinischen Behandlungssystems zu einem medizinischen Behandlungsinstrument des medizinischen Behandlungssystems und einen zweiten Kanal zum Abführen eines Aspirationsfluids vom medizinischen Behandlungsinstrument zur Konsole aufweist, wobei der erste und der zweite Kanal in einer Längserstreckungsrichtung des Schlauches parallel nebeneinander angeordnet sind, wobei die Kanäle jeweilige Wände aufweisen, welche in einem Verbindungsbereich des Schlauches miteinander verbunden sind. Weiterhin betrifft die Erfindung ein medizinisches Behandlungssystem, insbesondere ophthalmochirurgisches System zur Behandlung eines Auges, mit zumindest einem medizinischen Behandlungsinstrument, einer Konsole zum fluidtechnischen Versorgen des medizinischen Behandlungsinstruments in einem bestimmungsgemäßen Betrieb des medizinischen Behandlungssystems, und einem Schlauch zum Zuführen eines Irrigationsfluids von der Konsole zum medizinischen Behandlungsinstrument und zum Abführen eines Aspirationsfluids vom medizinischen Behandlungsinstrument zur Konsole.

Medizinische Behandlungssysteme, die Konsolen und medizinische Behandlungsinstrumente hierfür aufweisen, sowie Schläuche zum, insbesondere fluidtechnischen, Verbinden der Konsolen mit den medizinischen Behandlungsinstrumenten sind im Stand der Technik bekannt, sodass es diesbezüglich eines gesonderten druckschriftlichen Nachweises dem Grunde nach nicht bedarf. Beispielsweise zur Behandlung einer Augenlinsentrübung, in der Medizin auch als grauer Star bezeichnet, sind unterschiedliche chirurgische Techniken bekannt. Am weitesten verbreitet ist die Phakoemulsifikation, bei der eine dünne Hohlnadel in einen Kapselsack, in dem die Augenlinse angeordnet ist, eingeführt und zu Ultraschallschwingungen angeregt wird. Mittels der vibrierenden Hohlnadel kann die Linse emulsifiziert werden, wobei hierbei freigesetzte Linsenpartikel über eine Aspirationsleitung mittels einer Pumpe abgesaugt werden können. Dabei wird ein Spülfluid, auch Irrigationsfluid genannt, zugeführt. Die Linsenpartikel werden zusammen mit dem Fluid als Aspirationsfluid abgesaugt. Sobald die Linse vollständig emulsifiziert und entfernt ist, kann in den dann geleerten Kapselsack eine neue künstliche Linse eingesetzt werden. Hierdurch kann für den behandelten Patienten wieder ein gutes Sehvermögen erreicht werden.

Ein fortschrittliches ophthalmochirurgisches System, welches sich für die Phakoemulsifikation als besonders geeignet herausgestellt hat, offenbart zum Beispiel die DE 10 2016 201 297 B3. Bei diesem System werden jeweils zwei strömungstechnisch parallelgeschaltete Fluidpumpen für die Irrigation sowie auch für die Aspiration genutzt. Jede der Fluidpumpen weist eine Pumpkammer und eine mittels eines bevorzugt elastischen Trennelements von der Pumpkammer getrennte Antriebskammer auf. Die Antriebskammer wird für den bestimmungsgemäßen Betrieb der Fluidpumpe mit einem Antriebsfluid beaufschlagt, dessen Antriebsdruck zur Ausführung eines jeweiligen Pumphubs variiert wird. Dadurch verändert sich abhängig hiervon eine Auslenkungsposition des elastischen Trennelements, die sich entsprechend auf die Pumpkammer auswirkt. Die Pumpkammer ist mit dem jeweiligen Behandlungsfluid, beispielsweise dem Irrigationsfluid, dem Aspirationsfluid oder dergleichen, beaufschlagt. Durch geeignetes Steuern eines Einlass- und eines Auslassventils der Fluidpumpe, kann dann die Förderwirkung erreicht werden.

Eine Auslenkungsposition des elastischen Trennelements wird mittels eines der jeweiligen Fluidpumpe zugeordneten Auslenkungspositionssensors erfasst. Eine Steuereinrichtung des ophthalmochirurgischen Systems, insbesondere der Konsole steuert die Funktion der Fluidpumpe zumindest abhängig von einem Sensorsignal des Auslenkungspositionssensors und einem mittels eines Antriebsdrucksensors bereitgestellten Antriebsdrucksignal. Die Steuereinrichtung kann ergänzend beispielsweise das Einlass- und das Auslassventil entsprechend steuern.

Durch wechselseitiges Betätigen der jeweils zwei parallelgeschalteten Fluidpumpen kann während einer Operation ein Volumenstrom mit sehr geringen Schwankungen erreicht werden. Dadurch kann sich ein nahezu konstanter Augeninnendruck im Kapselsack einstellen. Solange ausreichend Irrigationsfluid zugeführt werden kann, kann das System auch während einer sehr lang andauernden Operation nahezu ohne Unterbrechung des Irrigationsfluidstroms betrieben werden.

Für die Versorgung des medizinischen Behandlungsinstruments, hier zum Beispiel ein Handstück, weist das medizinische Behandlungssystem in der Regel einen Schlauch auf, der die Konsole mit dem medizinischen Behandlungsinstrument insbesondere fluidtechnisch koppelt, sodass dem medizinischen Behandlungsinstrument einerseits das Irrigationsfluid zugeführt und andrerseits das Aspirationsfluid abgeführt werden kann. Hierzu können zweiflutige Schläuche vorgesehen sein, in denen für jedes der Fluide jeweils ein Kanal vorgesehen ist. Der jeweilige Kanal ist entlang seines gesamten Umfangs geschlossen ausgebildet. In der Regel sind zumindest die mit dem Behandlungsfluid in Kontakt stehenden Elemente in einer separaten Kassette angeordnet, die auswechselbar in einer Kassettenaufnahme der Konsole einsetzbar ist. Üblicherweise ist daher der konsolenseitige Anschluss des Schlauchs an der Kassette vorgesehen. In diesem Zusammenhang offenbart die EP 1 917 987 A2 Irrigations-/Aspirationssystem.

Für den bestimmungsgemäßen Betrieb ist es besonders bei ophthalmochirurgischen Systemen erwünscht, durch Regelung des Drucks des Irrigationsfluids und des Vakuums des Aspirationsfluids den Augeninnendruck, insbesondere im Kapselsack, möglichst konstant zu halten. Hierzu ist es erwünscht, den Druck des Behandlungsfluids möglichst genau zu kennen, damit eine entsprechend genaue Regelung dieses Drucks erreicht werden kann.

Es ist daher die Aufgabe der Erfindung, einen Schlauch sowie ein medizinisches Behandlungssystem dahingehend weiterzubilden, dass eine verbesserte Zuverlässigkeit im bestimmungsgemäßen Betrieb erreicht werden kann.

Als Lösung werden mit der Erfindung ein Schlauch für ein medizinisches Behandlungssystem sowie ein medizinisches Behandlungssystem, insbesondere ein ophthalmochirurgisches System zur Behandlung eines Auges, gemäß den unabhängigen Ansprüchen vorgeschlagen.

Vorteilhafte Weiterbildungen ergeben sich durch Merkmale der abhängigen Ansprüche.

In Bezug auf einen gattungsgemäßen Schlauch für ein medizinisches Behandlungssystem wird mit der Erfindung insbesondere vorgeschlagen, dass ein Werkstoff der Wand des zweiten Kanals eine größere Härte als der Werkstoff der Wand des ersten Kanals aufweist.

In Bezug auf ein gattungsgemäßes medizinisches Behandlungssystem wird mit der Erfindung insbesondere vorgeschlagen, dass der Schlauch gemäß der Erfindung ausgebildet ist.

Die Erfindung basiert unter anderem auf dem Gedanken, dass durch individuelle Wahl des Werkstoffs des Schlauchs im Bereich des jeweiligen der Kanäle die Funktionalität und die Stabilität des medizinischen Behandlungssystems insgesamt verbessert werden kann. Insbesondere bei ophthalmochirurgischen Systemen ist in der Regel vorgesehen, dass die Verbindung zwischen der Konsole beziehungsweise einer in der Konsole eingesetzten Kassette und dem medizinischen Behandlungsinstrument, hier insbesondere das Handstück, über einen Schlauch mit zwei Kanälen erfolgt, der auch als Twintube beziehungsweise Doppelschlauch bezeichnet wird. Ein Doppelschlauch ist nützlich, da er vom Bediener wie ein einziger Schlauch gehandhabt werden kann und somit eine hohe Bedienerfreundlichkeit erreicht wird. Es gibt aber auch Situationen, in denen ein Operateur zwei einzelne Schläuche benötigt, um bimanual arbeiten zu können. Dies kann zum Beispiel bei einer Notfalloperation wie einer anterioren Vitrektomie nach Ruptur des Kapselsackes oder beim Polieren der Kapselsackinnenwand oder beim Absaugen von Viskoelastikum der Fall sein. Dann muss ein Operateur schnell in der Lage sein, auf einer Länge von etwa 1 Meter den Doppelschlauch auftrennen zu können, um einen einzelnen Schlauch für das Irrigationsfluid und einen einzelnen Schlauch für das Aspirationsfluid nutzen zu können.

Der erfindungsgemäße Schlauch weist zwei Kanäle auf, die als Durchgangsöffnungen in Längsrichtung parallel zueinander beziehungsweise nebeneinander ausgebildet sind, wobei einer der Kanäle, zum Beispiel ein erster Kanal, dazu dient, das Irrigationsfluid von der Konsole beziehungsweise Kassette zum Handstück zuzuführen, und der andere der beiden Kanäle, zum Beispiel ein zweiter Kanal, dazu dient, das Aspirationsfluid vom Handstück beziehungsweise vom Auge zur Konsole beziehungsweise der in der Konsole eingesetzten Kassette zu führen beziehungsweise zu leiten. Die Erfindung ermöglicht es somit, ein möglichst geringes Surge-Verhalten bei gleichzeitig hoher Flexibilität und Ergonomie sowie guter Knickstabilität erreichen zu können. Das geringe Surge-Verhalten erreicht die Erfindung unter anderem dadurch, dass der Schlauch aus zwei einzelnen, in einem Verbindungsbereich verschweißten oder verklebten Schlauchelementen für die Kanäle gebildet ist, wobei für den Werkstoff der Wand des zweiten Kanals ein Werkstoff mit einer relativ großen Härte vorgesehen wird, wobei die Differenz in der Härte für den Werkstoff des ersten Kanals zur Härte für den Werkstoff des zweiten Kanals mindestens 10 ShA und maximal 15 ShA beträgt. Die Härte für den Werkstoff des zweiten Kanals ist in einem Bereich von 65 ShA bis 70 ShA gewählt. Für den Werkstoff der Wand des ersten Kanals, der für das Irrigationsfluid genutzt wird, wird ein Werkstoff eingesetzt, der eine Härte im Bereich von etwa 50 ShA bis 60 ShA aufweist. Dadurch kann ein Schlauch geschaffen werden, der quer zur Längserstreckung unterschiedliche Härteeigenschaften aufweist. Dabei liegt im Bereich des ersten Kanals, der für das Irrigationsfluid vorgesehen ist, eine kleinere Härte als im Bereich des zweiten Kanals vor, der für das Aspirationsfluid vorgesehen ist. Insbesondere in Bezug auf den zweiten für das Aspirationsfluid vorgesehenen Kanal kann daher ein möglichst geringes Surge-Verhalten bei gleichzeitig akzeptabler Flexibilität und Ergonomie und guter Knickstabilität erreicht werden. Die Stabilität des hierdurch erreichten zweiten Kanals erlaubt es ferner, auch bei einem kleinen Innendurchmesser noch eine ausreichende Strömung für das Aspirationsfluid erreichen zu können.

Von Bedeutung ist, dass die Differenz in der Härte für den Werkstoff des ersten Kanals zur Härte für den Werkstoff des zweiten Kanals mindestens 10 ShA und maximal 15 ShA beträgt. Der erfindungsgemäße Schlauch kann also so ausgebildet sein, dass die Differenz der Härten 13 ShA beträgt, indem zum Beispiel die Härte für den Werkstoff des ersten Kanals 57 ShA und die Härte für den Werkstoff des zweiten Kanals 70 ShA beträgt. Nur in diesem engen Differenzbereich der Härte von 10 ShA bis 15 ShA ist es möglich, eine gute Handhabbarkeit und eine ausreichende Knickstabilität für den ersten Kanal bei ausreichender Fähigkeit des zweiten Kanals, einem Surge entgegenzuwirken, zu schaffen, wobei sowohl der erste Kanal als auch der zweite Kanal mit einem kreisförmigen Innenquerschnitt versehen ist. Dabei liegt eine konstante Wanddicke entlang des gesamten Wandumfangs bis auf Ausnahme eines Verbindungsbereiches beider Wände vor. Sowohl der erste Kanal als auch der zweite Kanal benötigen keine Zonen zur Versteifung oder Verringerung der Steifigkeit durch einen Vorsprung oder eine Aussparung an ihrer jeweiligen Kanalwand. Die jeweilige Kanalwand ist bei dieser Ausführungsform im Querschnitt vollständig kreisförmig ausgebildet. Dies ist vorteilhaft, da somit eine gleichmäßige Strömung im jeweiligen Kanal erreichbar ist und keine Partikel an Vorsprüngen oder Ausnehmungen hängen bleiben und als Keime für ein Verstopfen eines Kanals wirken können. Da weder die erste Wand noch die zweite Wand entlang ihres Querschnitts einen Vorsprung oder eine Aussparung aufweisen, herrscht eine konstante Steifigkeit der Wände entlang ihrer gesamten Längserstreckung vor, sodass keine Gefahr für ein Einknicken besteht. Eine hohe Knickstabilität und eine hohe Sicherheit für eine gleichmäßige Strömung für das Irrigationsfluid und das Aspirationsfluid werden somit erreicht.

Der Schlauch kann somit als einstückiges Bauteil hergestellt sein, der zugleich die Kanäle für das Irrigationsfluid und das Aspirationsfluid bereitstellt. Der Schlauch kann zum Beispiel durch Extrudieren hergestellt werden, wobei während des Extrudierens zwei unterschiedliche Werkstoffe gemäß den gewünschten Härten eingesetzt werden. Dadurch kann natürlich auch die Zuverlässigkeit des medizinischen Behandlungssystems verbessert werden, weil der Schlauch bei der bestimmungsgemäßen Nutzung, die häufig eine manuelle Führung des medizinischen Behandlungsinstruments oder eine entsprechende Führung durch einen Roboter erfordert, einerseits klein und kompakt und daher leicht zu bedienen bleibt, wobei zugleich durch die erfindungsgemäße Ausgestaltung die Zuverlässigkeit und die Stabilität des Schlauchs verbessert werden kann. Insbesondere unerwünschte Knicke, die zu einer Störung bei der Nutzung führen können, können dadurch reduziert werden.

Ferner wird vorgeschlagen, dass die Dicke der Wand des ersten Kanals kleiner als die Dicke der Wand des zweiten Kanals ist. Dadurch kann für den zweiten Kanal eine erhöhte Knickstabilität auch bei hohem Saugdruck erreicht werden. Vorzugsweise ist die Dicke der Wand des ersten Kanals so gewählt, dass trotz der unterschiedlichen Werkstoffe zumindest in Bezug auf die Härte im Wesentlichen eine Stabilität erreicht werden kann, die etwa der Stabilität der Wand des das Aspirationsfluid führenden zweiten Wand entspricht.

Gemäß einer Weiterbildung wird vorgeschlagen, dass die Dicke der Wand des ersten Kanals mindestens 2,5 mm beträgt und/oder die Dicke der Wand des zweiten Kanals mindestens 2,6 mm beträgt. Hierdurch kann insbesondere für gängige Schlauchgrößen eine zuverlässige Stabilität erreicht werden.

Besonders vorteilhaft erweist es sich, wenn ein Innendurchmesser des ersten Kanals mindestens 3 mm beträgt und ein Innendurchmesser des zweiten Kanals mindestens 1,1 mm beträgt. Insbesondere für den Einsatz in einem ophthalmochirurgischen System können hierdurch geeignete fluide Strömungen im Schlauch erreicht werden, sodass ein bestimmungsgemäßer Betrieb realisiert werden kann. Je nach Bedarf können natürlich die Innendurchmesser auch größer gewählt sein, beispielsweise um Strömungswiderstände bei besonders langen Schläuchen reduzieren zu können. Vorzugsweise ist in einem solchen Fall jedoch auch vorgesehen, dass die jeweilige Dicke der Wand des ersten und/oder des zweiten Kanals entsprechend vergrößert gewählt ist.

Darüber hinaus wird vorgeschlagen, dass ein Außendurchmesser der Wand des ersten Kanals mindestens 5 mm, vorzugsweise etwa 5,5 mm, beträgt und ein Außendurchmesser der Wand des zweiten Kanals mindestens 4 mm, vorzugsweise etwa 4,1 mm, beträgt. Besonders die vorteilhaften Außendurchmesser ermöglichen es, handelsübliche Steckverbinder einsetzen zu können, beispielsweise einen handelsüblichen Luer-Konnektor. Dadurch kann die Erfindung auf einfache Weise bei bereits existierenden medizinischen Behandlungssystemen auch nachträglich eingesetzt werden, ohne aufwändige Anpassungen vornehmen zu müssen. Die Anwendung der Erfindung ist jedoch nicht hierauf beschränkt.

Bevorzugt liegt die Differenz des Außendurchmessers der Wand des ersten Kanals zu dem Außendurchmesser der Wand des zweiten Kanals im Bereich von 0,9 mm bis 1,6 mm. Bei einer kleineren Differenz der Außendurchmesser, also weniger als 0,9 mm, nimmt die Knickstabilität der Wand des ersten Kanals stark ab. Nur durch eine Differenz der Außendurchmesser im Bereich von 0,9 mm bis 1,6 mm wird eine hohe Knickstabilität der ersten Wand bei hoher Stabilität gegen einen Surge im zweiten Kanal erreicht, wobei gleichzeitig eine gute Ergonomie und Handhabbarkeit des gesamten Schlauches vorliegen.

Insgesamt kann vermieden werden, dass der Schlauch aufgrund der geringeren Härte der Wand des ersten Kanals zum Führen des Irrigationsfluids eine schlechtere Knickstabilität aufweist. Durch Erhöhen der Dicke dieser Wand kann sogar erreicht werden, dass der Schlauch diesbezüglich zumindest im Wesentlichen die gleiche Eigenschaft aufweist, sodass vorzugsweise eine nahezu homogene Eigenschaft, insbesondere in Bezug auf die Knickstabilität, erreicht werden kann.

Die für den erfindungsgemäßen Schlauch angegebenen Vorteile und Wirkungen gelten natürlich gleichermaßen auch für das mit dem erfindungsgemäßen Schlauch ausgerüstete medizinische Behandlungssystem und umgekehrt.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Fig. nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen.

In den Figuren zeigen:
- Fig. 1: eine schematische Blockdarstellung eines erfindungsgemäßen ophthalmochirurgischen Systems mit einer Konsole und einem mittels eines Schlauchs mit der Konsole gekoppelten Handstücks, und
- Fig. 2: eine schematische Schnittansicht des Schlauchs aus Fig. 1.

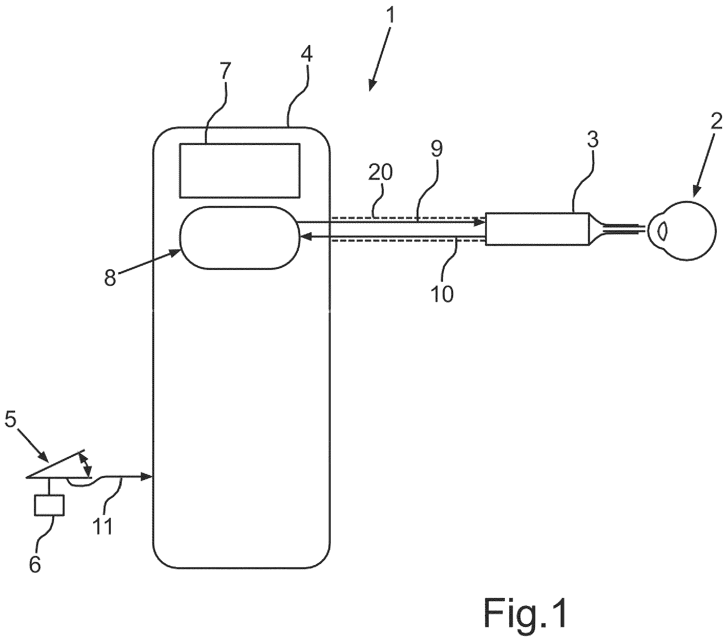

Fig. 1 zeigt in einer schematischen Blockdarstellung ein ophthalmochirurgisches System 1 als medizinisches Behandlungssystem, welches zur Behandlung eines Auges 2 dient.

Das ophthalmochirurgische System 1 weist ein medizinisches Behandlungsinstrument auf, welches vorliegend durch ein Handstück 3 gebildet ist. Ferner weist das ophthalmochirurgische System 1 eine Konsole 4 zum Anschließen und Betreiben des Handstücks 3 in einem bestimmungsgemäßen Betrieb auf. Weiterhin weist das ophthalmochirurgische System 1 eine Fußbedieneinheit 5 auf, die vorliegend als Fußpedal ausgebildet ist. Die Fußbedieneinheit 5 ist drahtlos mit der Konsole 4 koppelbar und weist einen elektrischen Energiespeicher 6 zum Versorgen der Fußbedieneinheit 5 mit elektrischer Energie zumindest während des bestimmungsgemäßen Betriebs des Handstücks 3 auf. Ferner weist die Konsole 4 eine Steuereinheit 7 sowie fluidische und elektrische Versorgungseinheiten 8 auf, die unter anderem dazu dienen, dem Handstück 3 über eine Irrigationsleitung 9 ein Irrigationsfluid zuzuführen und über eine Aspirationsleitung 10 ein Aspirationsfluid vom Handstück 3 abzuführen.

Vorliegend sind die Irrigationsleitung 9 und die Aspirationsleitung 10 durch einen einzigen Schlauch 20 bereitgestellt, der im Folgenden noch weiter erläutert werden wird. Die Irrigationsleitung 9 ist durch einen ersten Kanal 24 des Schlauchs 20 gebildet. Die Aspirationsleitung 10 ist durch einen zweiten Kanal 25 des Schlauchs 20 gebildet.

Die Fußbedieneinheit 5 ist vorliegend kabellos ausgebildet, weshalb sie zumindest während des bestimmungsgemäßen Betriebs vom elektrischen Energiespeicher 6 mit elektrischer Energie versorgt wird. Darüber hinaus ist die Fußbedieneinheit 5 im bestimmungsgemäßen Betrieb über eine drahtlose Kommunikationsverbindung 11 mit der Steuereinheit 7 in Kommunikationsverbindung, sodass hiermit ein Betriebszustand des Handstücks 3 zumindest teilweise eingestellt werden kann.

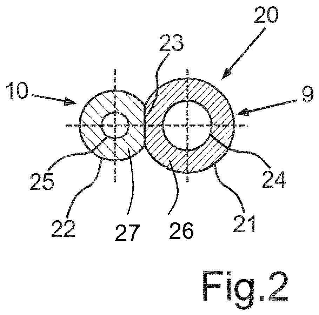

Fig. 2 zeigt eine schematische Schnittansicht des Schlauchs 20 gemäß Fig. 1. Aus Fig. 2 ist ersichtlich, dass der Schlauch zwei Kanäle 24, 25 aufweist, wobei ein erster Kanal 24 zum Zuführen des Irrigationsfluids von der Konsole 4 des medizinischen Behandlungssystems 1 zum medizinischen Behandlungsinstrument 3 des medizinischen Behandlungssystems 1 dient. Ein zweiter Kanal 25 dient zum Abführen des Aspirationsfluids vom medizinischen Behandlungsinstrument 3 zur Konsole 4. Der erste Kanal 24 und der zweite Kanal 25 sind quer zu einer Längserstreckungsrichtung des Schlauches 20 parallel nebeneinander angeordnet. Der Schlauch 20 stellt für den ersten Kanal 24 eine erste Wand 21 und für den zweiten Kanal 25 eine zweite Wand 22 mit einer vorgebbaren Dicke bereit. Die Wände 21, 22 sind in einem Verbindungsbereich 23 miteinander verbunden. Der Verbindungsbereich 23 erstreckt sich vorliegend etwa über eine gesamte Länge des Schlauches 20. In einer alternativen Ausgestaltung kann sich der Verbindungsbereich 23 auch lediglich über einen Teil der Länge des Schlauches 20 erstrecken.

In der vorliegenden Ausgestaltung ist vorgesehen, dass ein Werkstoff der zweiten Wand 22 des zweiten Kanals 25 eine größere Härte als der Werkstoff der ersten Wand 21 des ersten Kanals 24 aufweist. Vorliegend ist vorgesehen, dass der Werkstoff der ersten Wand 21 aus einem Polyvinylchlorid (PVC) gebildet ist, und zwar des Typs AM157/F 00. Der Werkstoff der zweiten Wand 22 ist vorliegend durch einen PVC des Typs AM171/F gebildet.

Dadurch kann erreicht werden, dass die erste Wand 21 eine Härte von etwa 57 ShA aufweist, wohingegen die zweite Wand 22 eine Härte von etwa 70 ShA aufweist. Je nach Bedarf kann die Härte jedoch auch variiert werden, um besondere Anforderungen besser berücksichtigen zu können.

Um die Eigenschaften des Schlauchs 20, insbesondere quer zu seiner Längserstreckung, hinsichtlich Knickstabilität zu verbessern, ist vorliegend vorgesehen, dass die Dicke der ersten Wand 21 des ersten Kanals 24 vorliegend etwa 2,5 mm beträgt. Je nach Bedarf kann diese Dicke jedoch auch größer gewählt sein. Die Dicke der zweiten Wand 22 des zweiten Kanals 25 beträgt vorliegend etwa 2,6 mm. Auch hier kann die Dicke unter Umständen auch größer gewählt sein.

Die Dicke der ersten Wand 21 des ersten Kanals 24 kann auch kleiner als die Dicke der zweiten Wand 22 des zweiten Kanals 25 sein. Durch die größere Dicke der zweiten Wand 21 kann auch in diesem Bereich eine vergrößerte Steifigkeit erreicht werden, sodass entsprechend vergrößerte Knickstabilität erreicht werden kann.

Um für den bestimmungsgemäßen Betrieb des ophthalmochirurgischen Systems einen ausreichenden Strömungsquerschnitt bereitstellen zu können, ist vorliegend vorgesehen, dass ein Innendurchmesser des ersten Kanals 24 etwa 3 mm beträgt. Je nach Bedarf kann dieser Innendurchmesser auch größer gewählt sein. Ein Innendurchmesser des zweiten Kanals 25 beträgt dagegen vorliegend etwa 1,5 mm. Auch hier kann bedarfsweise der Innendurchmesser auch größer gewählt sein. Von Bedeutung ist, dass der Innendurchmesser des ersten Kanals 24 immer größer als der Innendurchmesser des zweiten Kanals 25 ist.

Ein Außendurchmesser der Wand 21 des ersten Kanals 24 beträgt vorliegend etwa 5,5 mm, wohingegen ein Außendurchmesser der Wand 22 des zweiten Kanals 25 vorliegend etwa 4,1 mm beträgt. Je nach Bedarf kann der Außendurchmesser der Wände 21, 22 auch variabel, insbesondere größer gewählt sein.

Im Verbindungsbereich 23 sind die beiden Wände 21, 22 stoffschlüssig miteinander verbunden. Dies kann beispielsweise unmittelbar bei einem Extrudieren des Schlauches 20 erfolgen. Der Schlauch 20 kann jedoch auch aus zwei einzelnen Schlauchelementen 26, 27 für die Kanäle 24, 25 gebildet sein. Die Schlauchelemente 26, 27 sind dann zum Beispiel nicht koextrudiert, sondern einzeln extrudiert, wobei sie nachträglich miteinander verschweißt oder verklebt werden. Im Verbindungsbereich 23 können sich die Wände 21, 22 zumindest teilweise überlappen. Dadurch kann der Werkstoff der Wände 21, 22 zumindest teilweise auch für die Verbindung der beiden Kanäle 24, 25 genutzt werden, um den Schlauch 20 auszubilden. Einzeln extrudierte und anschließend verklebte oder verschweißte Schlauchelemente 26, 27 haben den Vorteil, dass bei Bedarf ein nachträgliches Trennen der Schlauchelemente 26, 27 im Verbindungsbereich 23 in wieder zwei einzelne Schlauchelemente 26, 27 sicher und zuverlässig möglich ist und die Kanäle 24, 25 unverletzt erhalten bleiben.

Die Ausführungsbeispiele dienen ausschließlich der Erläuterung der Erfindung und sollen diese nicht beschränken.

## Patentansprüche

1. Schlauch (20) für ein medizinisches Behandlungssystem, insbesondere ein ophthalmochirurgisches System (1) zur Behandlung eines Auges (2), wobei der Schlauch (20) einen ersten Kanal (24) zum Zuführen eines Irrigationsfluids von einer Konsole (4) des medizinischen Behandlungssystems zu einem medizinischen Behandlungsinstrument (3) des medizinischen Behandlungssystems und einen zweiten Kanal (25) zum Abführen eines Aspirationsfluids vom medizinischen Behandlungsinstrument (3) zur Konsole (4) aufweist, wobei der erste Kanal (24) und der zweite Kanal (25) in einer Längserstreckungsrichtung des Schlauches (20) parallel nebeneinander angeordnet sind, wobei der erste Kanal (24) eine erste Wand (21) und der zweite Kanal (25) eine zweite Wand (22) aufweisen, wobei ein Werkstoff der zweiten Wand (22) des zweiten Kanals (25) eine größere Härte als der Werkstoff der ersten Wand (21) des ersten Kanals (24) aufweist,
**dadurch gekennzeichnet, dass**
der Schlauch (20) aus zwei einzelnen, in einem Verbindungsbereich (23) verschweißten oder verklebten Schlauchelementen (26, 27) für die Kanäle (24, 25) gebildet ist, wobei die Härte für den Werkstoff des ersten Kanals (24) in einem Bereich von 50 ShA bis 60 ShA gewählt ist, wobei die Härte für den Werkstoff des zweiten Kanals (25) in einem Bereich von 65 ShA bis 70 ShA gewählt ist, und wobei die Differenz in der Härte für den Werkstoff des ersten Kanals (24) zur Härte für den Werkstoff des zweiten Kanals (25) mindestens 10 ShA und maximal 15 ShA beträgt.

2. Schlauch nach Anspruch 1,
**dadurch gekennzeichnet, dass**
eine Dicke der Wand (21) des ersten Kanals (24) kleiner als eine Dicke der Wand (22) des zweiten Kanals (25) ist.

3. Schlauch nach Anspruch 2,
**dadurch gekennzeichnet, dass**
die Dicke der Wand (21) des ersten Kanals (24) mindestens 2,5 mm beträgt und/oder die Dicke der Wand (22) des zweiten Kanals (25) mindestens 2,6 mm beträgt.

4. Schlauch nach einem der vorhergehenden Ansprüche,
**dadurch gekennzeichnet, dass**
ein Innendurchmesser des ersten Kanals (24) mindestens 3 mm beträgt und ein Innendurchmesser des zweiten Kanals (25) mindestens 1,1 mm beträgt.

5. Schlauch nach einem der vorhergehenden Ansprüche,
**dadurch gekennzeichnet, dass**
ein Außendurchmesser des ersten Kanals (24) mindestens 5,5 mm beträgt und ein Außendurchmesser des zweiten Kanals (25) mindestens 4,1 mm beträgt, wobei die Differenz des Außendurchmessers der Wand des ersten Kanals (24) zu dem Außendurchmesser der Wand des zweiten Kanals (25) im Bereich von 0,9 mm bis 1,6 mm beträgt.

6. Medizinisches Behandlungssystem, insbesondere ophthalmochirurgisches System (1) zur Behandlung eines Auges (2), mit zumindest:
- einem medizinischen Behandlungsinstrument (3),
- einer Konsole (4) zum fluidtechnischen Versorgen des medizinischen Behandlungsinstruments (3) in einem bestimmungsgemäßen Betrieb des medizinischen Behandlungssystems, und
- einem Schlauch (20) zum Zuführen eines Irrigationsfluids von der Konsole (4) zum medizinischen Behandlungsinstrument (3) und zum Abführen eines Aspirationsfluids vom medizinischen Behandlungsinstrument (3) zur Konsole (4), **dadurch gekennzeichnet, dass**
der Schlauch (20) nach einem der vorhergehenden Ansprüche ausgebildet ist.

## Claims

1. Tube (20) for a medical treatment system, in particular an ophthalmosurgical system (1) for treatment of an eye (2), wherein the tube (20) has a first channel (24) for feeding an irrigation fluid from a console (4) of the medical treatment system to a medical treatment instrument (3) of the medical treatment system and a second channel (25) for discharging an aspiration fluid from the medical treatment instrument (3) to the console (4), wherein the first channel (24) and the second channel (25) are arranged in parallel next to each other in a direction of longitudinal extent of the tube (20), wherein the first channel (24) has a first wall (21) and the second channel (25) has a second wall (22), wherein a material of the second wall (22) of the second channel (25) has a greater hardness than the material of the first wall (21) of the first channel (24),
**characterized in that**
the tube (20) is formed from two individual tube elements (26, 27) for the channels (24, 25), said tube elements (26, 27) being welded or adhesively bonded in a connection region (23), wherein the hardness of the material for the first channel (24) is chosen in a range of 50 ShA to 60 ShA, wherein the hardness of the material for the second channel (25) is chosen in a range of 65 ShA to 70 ShA, and wherein the difference in hardness between the hardness of the material for the first channel (24) and the hardness of the material for the second channel (25) is at least 10 ShA and at most 15 ShA.

2. Tube according to Claim 1,
**characterized in that**
a thickness of the wall (21) of the first channel (24) is smaller than a thickness of the wall (22) of the second channel (25).

3. Tube according to Claim 2,
**characterized in that**
the thickness of the wall (21) of the first channel (24) is at least 2.5 mm and/or the thickness of the wall (22) of the second channel (25) is at least 2.6 mm.

4. Tube according to any one of the preceding claims,
**characterized in that**
an internal diameter of the first channel (24) is at least 3 mm, and an internal diameter of the second channel (25) is at least 1.1 mm.

5. Tube according to any one of the preceding claims,
**characterized in that**
an external diameter of the first channel (24) is at least 5.5 mm and an external diameter of the second channel (25) is at least 4.1 mm, wherein the difference between the external diameter of the wall of the first channel (24) and the external diameter of the wall of the second channel (25) is in the range of 0.9 mm to 1.6 mm.

6. Medical treatment system, in particular an ophthalmosurgical system (1) for treatment of an eye (2), having at least:
- a medical treatment instrument (3),
- a console (4) for supplying fluid to the medical treatment instrument (3) in a defined operating mode of the medical treatment system, and
- a tube (20) for feeding an irrigation fluid from the console (4) to the medical treatment instrument (3) and for discharging an aspiration fluid from the medical treatment instrument (3) to the console (4),
**characterized in that**
the tube (20) is designed according to any one of the preceding claims.

## Revendications

1. Tuyau (20) pour un système de traitement médical, notamment un système de chirurgie ophtalmique (1) pour le traitement d'un œil (2), le tuyau (20) présentant un premier canal (24) pour acheminer un fluide d'irrigation depuis une console (4) du système de traitement médical vers un instrument de traitement médical (3) du système de traitement médical, et un deuxième canal (25) pour évacuer un fluide d'aspiration depuis l'instrument de traitement médical (3) vers la console (4), le premier canal (24) et le deuxième canal (25) étant agencés parallèlement l'un à côté de l'autre dans une direction d'extension longitudinale du tuyau (20), le premier canal (24) présentant une première paroi (21) et le deuxième canal (25) présentant une deuxième paroi (22), un matériau de la deuxième paroi (22) du deuxième canal (25) présentant une dureté supérieure à celle du matériau de la première paroi (21) du premier canal (24),
**caractérisé en ce que**
le tuyau (20) est formé de deux éléments de tuyau (26, 27) individuels, soudés ou collés dans une zone de liaison (23) pour les canaux (24, 25), la dureté du matériau du premier canal (24) étant choisie dans une plage de 50 ShA à 60 ShA, la dureté du matériau du deuxième canal (25) étant choisie dans une plage de 65 ShA à 70 ShA, et la différence entre la dureté du matériau du premier canal (24) et la dureté du matériau du deuxième canal (25) étant d'au moins 10 ShA et d'au maximum 15 ShA.

2. Tuyau selon la revendication 1,
**caractérisé en ce que**
une épaisseur de la paroi (21) du premier canal (24) est inférieure à une épaisseur de la paroi (22) du deuxième canal (25).

3. Tuyau selon la revendication 2,
**caractérisé en ce que**
l'épaisseur de la paroi (21) du premier canal (24) est d'au moins 2,5 mm et/ou l'épaisseur de la paroi (22) du deuxième canal (25) est d'au moins 2,6 mm.

4. Tuyau selon l'une quelconque des revendications précédentes,
**caractérisé en ce que**
un diamètre intérieur du premier canal (24) est d'au moins 3 mm et un diamètre intérieur du deuxième canal (25) est d'au moins 1,1 mm.

5. Tuyau selon l'une quelconque des revendications précédentes,
**caractérisé en ce que**
un diamètre extérieur du premier canal (24) est d'au moins 5,5 mm et un diamètre extérieur du deuxième canal (25) est d'au moins 4,1 mm, la différence entre le diamètre extérieur de la paroi du premier canal (24) et le diamètre extérieur de la paroi du deuxième canal (25) étant dans la plage de 0,9 mm à 1,6 mm.

6. Système de traitement médical, notamment système de chirurgie ophtalmique (1) pour le traitement d'un œil (2), avec au moins :
- un instrument de traitement médical (3),
- une console (4) pour l'alimentation en fluide de l'instrument de traitement médical (3) lors d'une utilisation conforme du système de traitement médical, et
- un tuyau (20) pour acheminer un fluide d'irrigation depuis la console (4) vers l'instrument de traitement médical (3) et pour évacuer un fluide d'aspiration depuis l'instrument de traitement médical (3) vers la console (4),
**caractérisé en ce que**
le tuyau (20) est réalisé selon l'une quelconque des revendications précédentes.
